# EUROPEAN PATENT APPLICATION

(11) **EP 3 167 801 A1**
(43) Date of publication of application: **17.05.2017**
(21) Application number: 15818422.6
(22) Date of filing: 25.06.2015
(51) Int. Cl.: A61B 5/11, G01S 13/36

(54) **WIRELESS SENSOR APPARATUS**

(30) Priority: 10.07.2014 JP 2014141922
(71) Applicant: Alps Electric Co., Ltd., Tokyo 145-8501 (JP)
(72) Inventor: OTAKI, Yukio, Tokyo 145-8501 (JP); KIM, Junghyun, Tokyo 145-8501 (JP)
(74) Representative: Klunker . Schmitt-Nilson . Hirsch
(86) International application number: PCT/JP2015/068379
(87) International publication number: WO 2016/006468

(57) **Abstract**

There is provided a wireless sensor device capable of suppressing a reduction in detection sensitivity even if the distance between the wireless sensor device and a detection target is changed. A wireless sensor device 1 includes: a sensor unit 10 that generates a detection signal based on a transmission signal emitted to a detection target 30 and a reflection signal from the detection target of the transmission signal; and a signal processing unit 20 that performs signal processing on the detection signal generated by the sensor unit 10 . The sensor unit 10 generates a first detection signal Sa1 corresponding to a first phase state, which is a phase state of the transmission signal or the reflection signal, and a second detection signal Sa2 corresponding to a second phase state in which a phase of the transmission signal or the reflection signal is different from that in the first phase state. The signal processing unit 20 detects phase information based on the first detection signal Sa1 and second detection signal Sa2, and performs predetermined signal processing based on the detected phase information.

## Description

### TECHNICAL FIELD

The present invention relates to a wireless sensor device, and in particular, to a wireless sensor device for detecting the movement of a detection target based on a transmission signal emitted to the detection target and a reflection signal from the detection target of the transmission signal.

### BACKGROUND ART

A wireless sensor device has been put into practical use which includes a sensor unit and a signal processing unit and detects the movement of a detection target based on a transmission signal emitted to the detection target and a reflection signal from the detection target of the transmission signal. Such a wireless sensor device is used as a biological sensor for detecting a movement relevant to biological information, such as the breathing or heart beat movement of a person.

As a known wireless sensor device, a radio frequency sensor (wireless sensor device) according to PTL 1 has been proposed. Fig. 12 is an explanatory diagram showing the configuration of a radio frequency sensor 410 according to PTL 1.

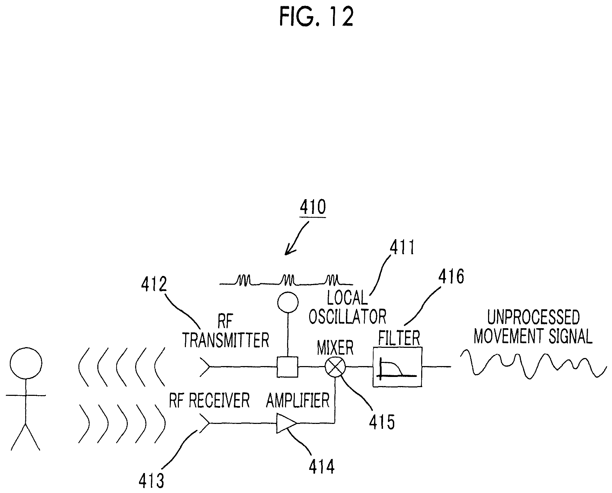

As shown in Fig. 12, the radio frequency sensor 410 includes a local oscillator 411, an RF transmitter 412, an RF receiver 413, an amplifier 414, a mixer 415, and a low pass filter 416. The radio frequency sensor 410 further includes an arithmetic control circuit (not shown).

The local oscillator 411 generates a high-frequency signal. The RF transmitter 412 emits the high-frequency signal generated by the local oscillator 411 toward a subject (detection target), such as a person, using an antenna provided therein. The RF receiver 413 receives a reflection signal from the subject (detection target), of the transmission signal emitted from the RF transmitter 412, using an antenna provided therein. The amplifier 414 amplifies the reflection signal received by the RF receiver 413.

The mixer 415 mixes a part of the transmission signal and the amplified reflection signal. The low pass filter 416 removes a noise component of the output signal of the mixer 415. The output signal of the low pass filter 416 is an unprocessed sensor signal including information regarding a movement, such as the breathing or heart beat movement of the subject. The unprocessed sensor signal is a signal corresponding to the phase difference between the transmission signal and the reflection signal.

The radio frequency sensor 410 further includes an arithmetic control circuit (not shown). In addition, the arithmetic control circuit controls various circuits, such as the local oscillator 411, the RF transmitter 412, the RF receiver 413, the amplifier 414, the mixer 415, and the low pass filter 416. In addition, the arithmetic control circuit receives the unprocessed sensor signal from the low pass filter 416, and calculates movement information regarding a movement, such as the breathing or heart beat movement of the subject, based on the received unprocessed sensor signal. In this manner, the radio frequency sensor 410 detects the movement of the subject.

### CITATION LIST

### PATENT LITERATURE

PTL 1: PCT Japanese Translation Patent Publication No. 2009-538720

### DISCLOSURE OF THE INVENTION

### PROBLEMS TO BE SOLVED BY THE INVENTION

In such a wireless sensor device, if the distance between the wireless sensor device and the detection target is changed due to the movement of the detection target while the movement of the detection target is being detected, the phase difference between the transmission signal and the reflection signal is changed corresponding to the distance change. In addition, in such a wireless sensor device, a signal for detecting the movement of the detection target is generated using the phase difference between the transmission signal and the reflection signal. For this reason, depending on the phase difference between the transmission signal and the reflection signal, detection sensitivity when detecting the movement of the detection target is greatly reduced. Accordingly, there have been cases where it is difficult to detect the movement of the detection target.

Even in a case where the detection sensitivity is not reduced greatly, if the distance between the wireless sensor device and the detection target is frequently changed and the phase difference between the transmission signal and the reflection signal is changed corresponding to the distance change, the phase or electric potential of a signal for detecting the movement of the detection target is also changed due to the influence. For this reason, there has been a possibility that the analysis of the movement of the detection target will become complicated. Also in this case, the detection sensitivity when detecting the movement of the detection target is reduced.

The present invention has been made in view of such conventional circumstances, and it is an object of the present invention to provide a wireless sensor device capable of suppressing a reduction in detection sensitivity even if the distance between the wireless sensor device and a detection target is changed.

### MEANS FOR SOLVING THE PROBLEMS

In order to solve the problem, a wireless sensor device according to a first aspect of the present invention is a wireless sensor device including: a sensor unit that generates a detection signal based on a transmission signal emitted to a detection target and a reflection signal from the detection target of the transmission signal; and a signal processing unit that performs signal processing on the detection signal generated by the sensor unit. The sensor unit generates a first detection signal corresponding to a first phase state, which is a phase state of the transmission signal or the reflection signal, and a second detection signal corresponding to a second phase state in which a phase of the transmission signal or the reflection signal is different from that in the first phase state. The signal processing unit detects phase information based on the first detection signal and the second detection signal, and performs predetermined signal processing based on the detected phase information.

In the wireless sensor device of the configuration, the sensor unit generates a first detection signal corresponding to the predetermined first phase state and a second detection signal corresponding to the second phase state in which the phase of the transmission signal or the reflection signal is different from that in the first phase state. Therefore, even if the distance between the wireless sensor device and the detection target is changed to reduce the detection sensitivity in one of the first and second phase states, it is possible to create a state with good detection sensitivity by performing switching to the other phase state.

In addition, since the first detection signal and the second detection signal are signals corresponding to two different phase states, the first detection signal and the second detection signal are signals suitable for detecting phase information. In addition, the signal processing unit detects phase information based on the first detection signal and the second detection signal, and performs predetermined signal processing based on the detected phase information. Therefore, even in a case where the distance between the wireless sensor device and the detection target is frequently changed and the phase difference between the transmission signal and the reflection signal is changed, it is possible to detect the phase information based on the first detection signal and the second detection signal and correct the signal based on the detected phase information. As a result, it is possible to suppress a reduction in detection sensitivity according to a change in the distance between the wireless sensor device and the detection target.

In the wireless sensor device according to a second aspect of the present invention, the second phase state may be a phase state in which the phase of the reflection signal is different by π/2 (radian) from that in the first phase state, and the signal processing unit may calculate a phase angle in orthogonal coordinates based on the first detection signal and the second detection signal and correct a phase of a signal based on the calculated phase angle.

In the wireless sensor device of the configuration, the second phase state is a phase state in which the phase of the reflection signal is different by π/2 (radian) from that in the first phase state. Accordingly, by setting the first detection signal and the second detection signal corresponding to such two phase states as two coordinate components in the orthogonal coordinates, it is possible to easily calculate the phase angle in the orthogonal coordinates based on the first detection signal and the second detection signal. In addition, the signal processing unit calculates the phase angle in the orthogonal coordinates based on the first detection signal and the second detection signal, and corrects the phase of the signal based on the calculated phase angle. Therefore, by correcting the phase of the signal, the signal before correction can be converted into a signal that is hardly influenced by changes in the phase angle. As a result, since the influence of the change of the signal due to the change in the phase difference between the transmission signal and the reflection signal can be further reduced, it is possible to further suppress a reduction in detection sensitivity.

In the wireless sensor device according to a third aspect of the present invention, the second phase state may be a phase state in which the phase of the reflection signal is delayed by π/2 (radian) with respect to that in the first phase state, and the signal processing unit may calculate the phase angle as an arc tangent function for a value obtained by dividing first numerical information by second numerical information using the first numerical information corresponding to a mixed signal of the first detection signal and the second detection signal and the second numerical information corresponding to a mixed signal of the first detection signal and the first detection signal.

In the wireless sensor device of the configuration, the second phase state is a phase state in which the phase of the reflection signal is delayed by π/2 (radian) with respect to that in the first phase state. Accordingly, the first detection signal corresponding to the first phase state can be used as a cosine component in the orthogonal coordinates, and the second detection signal corresponding to the second phase state can be used as a sine component in the orthogonal coordinates.

Since the first numerical information is a signal corresponding to the mixed signal of the first detection signal and the second detection signal, the first numerical information is a value proportional to the product of the first detection signal and the second detection signal, that is, a value proportional to the product of the cosine function and sine function for the phase angle. In addition, since the second numerical information is a signal corresponding to the mixed signal of the first detection signal and the first detection signal, the second numerical information is a value proportional to the square of the first detection signal, that is, a value proportional to the square of the cosine function for the phase angle. Therefore, the phase angle can be calculated using a simple calculation expression of an arc tangent function for a value obtained by dividing the first numerical information by the second numerical information. As a result, it becomes easy to calculate the phase angle.

In the wireless sensor device according to a fourth aspect of the present invention, the second phase state may be a phase state in which the phase of the reflection signal is delayed by π/2 (radian) with respect to that in the first phase state, and the signal processing unit may calculate the phase angle as an arc tangent function for a value obtained by dividing third numerical information by first numerical information using the first numerical information corresponding to a mixed signal of the first detection signal and the second detection signal and the third numerical information corresponding to a mixed signal of the second detection signal and the second detection signal.

In the wireless sensor device of the configuration, the second phase state is a phase state in which the phase of the reflection signal is delayed by π/2 (radian) with respect to that in the first phase state. Accordingly, the first detection signal corresponding to the first phase state can be used as a cosine component in the orthogonal coordinates, and the second detection signal corresponding to the second phase state can be used as a sine component in the orthogonal coordinates.

Since the first numerical information is a signal corresponding to the mixed signal of the first detection signal and the second detection signal, the first numerical information is a value proportional to the product of the first detection signal and the second detection signal, that is, a value proportional to the product of the cosine function and sine function for the phase angle. In addition, since the third numerical information is a signal corresponding to the mixed signal of the second detection signal and the second detection signal, the third numerical information is a value proportional to the square of the second detection signal, that is, a value proportional to the square of the sine function for the phase angle. Therefore, the phase angle can be calculated using a simple calculation expression of an arc tangent function for a value obtained by dividing the third numerical information by the first numerical information. As a result, it becomes easy to calculate the phase angle.

In the wireless sensor device according to a fifth aspect of the present invention, the second phase state may be a phase state in which the phase of the reflection signal is delayed by π/2 (radian) with respect to that in the first phase state, and the signal processing unit may calculate a first calculation value of the phase angle as an arc tangent function for a value obtained by dividing first numerical information by second numerical information and calculate a second calculation value of the phase angle as an arc tangent function for a value obtained by dividing third numerical information by the first numerical information using the first numerical information corresponding to a mixed signal of the first output signal and the second output signal, the second numerical information corresponding to a mixed signal of the first output signal and the first output signal, and the third numerical information corresponding to a mixed signal of the second output signal and the second output signal, and may set one value selected from the first and second calculation values or an average value of the first and second calculation values as a calculation value of the phase angle.

In the wireless sensor device of the configuration, the second phase state is a phase state in which the phase of the reflection signal is delayed by π/2 (radian) with respect to that in the first phase state. Accordingly, the first detection signal corresponding to the first phase state can be used as a cosine component in the orthogonal coordinates, and the second detection signal corresponding to the second phase state can be used as a sine component in the orthogonal coordinates.

In addition, the phase angle can be calculated using two calculation methods of a calculation method for calculating the phase angle as an arc tangent function for a value obtained by dividing the first numerical information by the second numerical information and a calculation method for calculating the phase angle as an arc tangent function for a value obtained by dividing the third numerical information by the first numerical information. As a result, since it is possible to calculate the phase angle using simple mathematical expressions, it becomes easy to calculate the phase angle.

In addition, in the wireless sensor device of the configuration, the first calculation value of the phase angle is calculated as the arc tangent function for the value obtained by dividing the first numerical information by the second numerical information, and the second calculation value of the phase angle is calculated as the arc tangent function for the value obtained by dividing the third numerical information by the first numerical information. One value selected from the first and second calculation values or the average value of the first and second calculation values is set as a calculation value of the phase angle. Therefore, even in a case where the calculation accuracy of one of the first and second calculation values is reduced, it is possible to suppress a reduction in detection sensitivity by using the other one as a calculation value of the phase angle or by using the average value of the first and second calculation values as a calculation value of the phase angle.

In the wireless sensor device according to a sixth aspect of the present invention, the sensor unit may include: a transmitting and receiving antenna for emitting the transmission signal and receiving the reflection signal; a signal generating circuit that generates the transmission signal; a phase shifter that generates the first and second phase states; a first detection circuit to which a part of the transmission signal and the reflection signal corresponding to the first phase state are input; and a second detection circuit to which a part of the transmission signal and the reflection signal corresponding to the second phase state are input. The signal processing unit may include: a phase detection circuit to which the first detection signal and the second detection signal are input; and a signal processing circuit to which the first detection signal, the second detection signal, and the phase information are input.

In the wireless sensor device of the configuration, the sensor unit can easily generate the first detection signal corresponding to the first phase state and the second detection signal corresponding to the second phase state using the phase shifter, the first detection circuit, and the second detection circuit. In addition, the signal processing unit can easily perform the detection of phase information and signal processing using the phase detection circuit and the signal processing circuit. As a result, in the wireless sensor device of the configuration, it is possible to easily detect the movement of the detection target.

In the wireless sensor device according to a seventh aspect of the present invention, a plurality of the signal processing units may be provided, the signal processing unit may include a first filter, which has a predetermined pass band and to which the first detection signal is input, and a second filter, which has the predetermined pass band and to which the second detection signal is input, and the predetermined pass band may be a different band for each of the signal processing units.

In the wireless sensor device of the configuration, it is possible to detect a plurality of different pieces of movement information from the movement of the detection target using a plurality of signal processing units. In the case of detecting a plurality of pieces of movement information, there is a case in which frequencies suitable for detection are different for each of the pieces of movement information. In the wireless sensor device of the configuration, however, the pass band of the first filter and the pass band of the second filter are different bands for each signal processing unit. Therefore, it is possible to extract a frequency component suitable for the detection of movement for each signal processing unit from the detection signal output from the sensor unit. As a result, in the wireless sensor device of the configuration, it is possible to efficiently detect a plurality of different movements of the detection target.

According to the present invention, it is possible to provide a wireless sensor device capable of suppressing a reduction in detection sensitivity even if the distance between the wireless sensor device and a detection target is changed.

### BRIEF DESCRIPTION OF THE DRAWINGS

Figs. 1A and 1B are explanatory diagrams showing the configuration of a wireless sensor device 1 according to a first embodiment of the present invention.
Fig. 2 is an explanatory diagram showing the configuration of a sensor unit 10 shown in Figs. 1A and 1B in more detail.
Fig. 3 is an explanatory diagram showing the configuration of a signal processing unit 20 shown in Figs. 1A and 1B in more detail.
Fig. 4 is an explanatory diagram showing the phase state of a reflection signal according to the first embodiment of the present invention.
Figs . 5A and 5B are explanatory diagrams regarding a phase correction method according to the first embodiment of the present invention.
Figs. 6A and 6B are explanatory diagrams showing the configuration of a wireless sensor device 101 according to a second embodiment of the present invention.
Fig. 7 is an explanatory diagram showing the configuration of a signal processing unit 120 shown in Figs. 6A and 6B in more detail.
Figs. 8A and 8B are explanatory diagrams showing the configuration of a wireless sensor device 201 according to a third embodiment of the present invention.
Fig. 9 is an explanatory diagram showing the configuration of a signal processing unit 220 shown in Figs. 8A and 8B in more detail.
Fig. 10 is an explanatory diagram showing the configuration of a wireless sensor device 301 according to a fourth embodiment of the present invention.
Fig. 11 is an explanatory diagram showing the configuration of a signal processing unit 320 shown in Fig. 10 in more detail.
Fig. 12 is an explanatory diagram showing the configuration of a radio frequency sensor 410 according to PTL 1.

### BEST MODE FOR CARRYING OUT THE INVENTION

### [First embodiment]

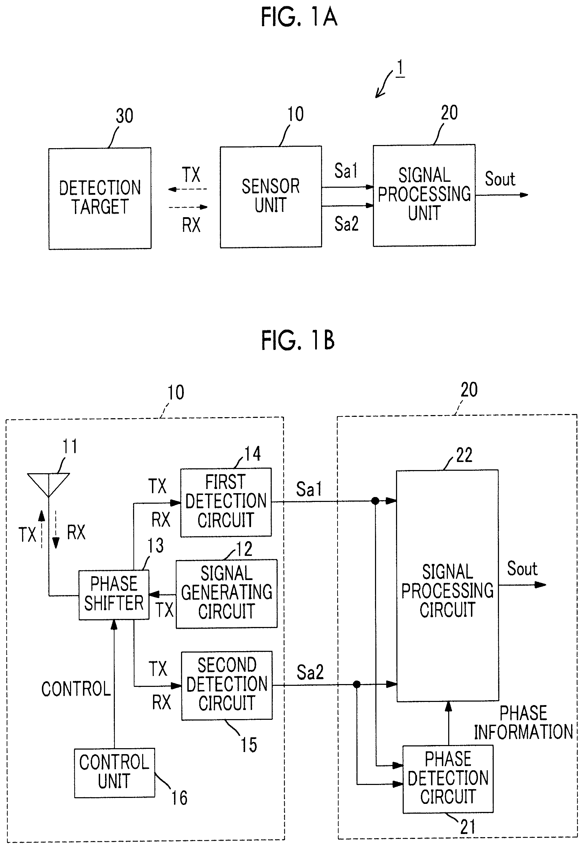

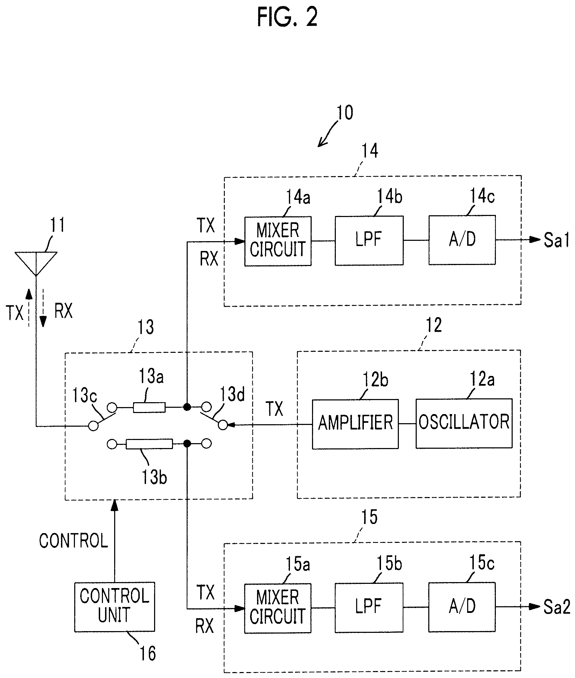

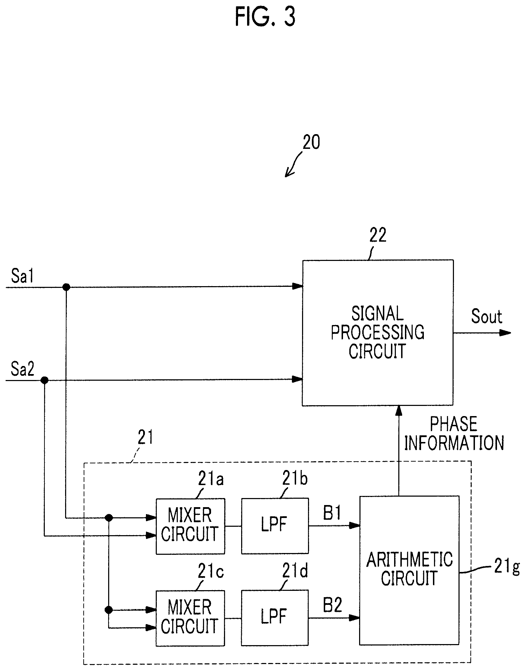

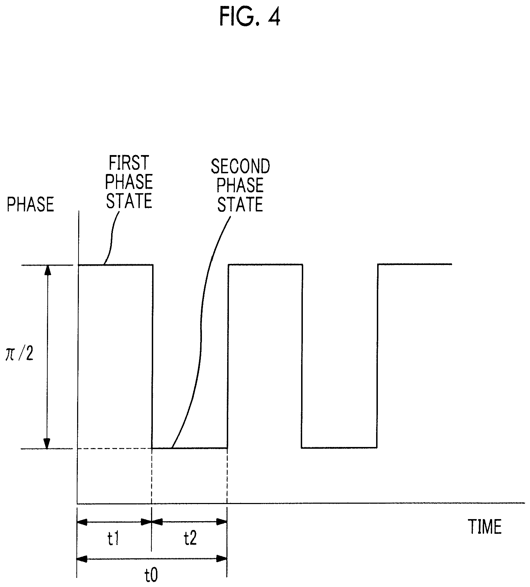

Hereinafter, a first embodiment of the present embodiment will be described with reference to the diagrams. First, the configuration of a wireless sensor device according to the first embodiment of the present invention will be described with reference to Figs. 1A to 4 . Figs. 1A and 1B are explanatory diagrams showing the configuration of a wireless sensor device 1 according to the first embodiment of the present invention. Fig. 1A is an explanatory diagram showing the overall configuration of the wireless sensor device 1, and Fig. 1B is an explanatory diagram showing the configuration of the wireless sensor device 1 in more detail. Fig. 2 is an explanatory diagram showing the configuration of a sensor unit 10 shown in Figs. 1A and 1B in more detail. Fig. 3 is an explanatory diagram showing the configuration of a signal processing unit 20 shown in Figs. 1A and 1B in more detail. Fig. 4 is an explanatory diagram showing the phase state of a reflection signal according to the first embodiment of the present invention. In Fig. 4, the horizontal axis indicates time, and the vertical axis indicates the phase of a reflection signal RX.

As shown in Figs. 1A and 1B, the wireless sensor device 1 includes the sensor unit 10 and the signal processing unit 20. In addition, the wireless sensor device 1 detects the movement of a detection target 30 based on a transmission signal TX emitted to the detection target 30 and the reflection signal RX from the detection target 30 of the transmission signal. Such a wireless sensor device 10 is used as a biological sensor for detecting a movement relevant to biological information, such as the breathing or heart beat movement of a person.

As shown in Figs. 1A and 1B, the sensor unit 10 includes a transmitting and receiving antenna 11, a signal generating circuit 12, a phase shifter 13, a first detection circuit 14, a second detection circuit 15, and a control circuit 16.

The transmitting and receiving antenna 11 is an antenna for emitting the transmission signal TX and receiving the reflection signal RX. The sensor unit 10 emits the transmission signal TX to the detection target 30 as an electromagnetic wave signal using the transmitting and receiving antenna 11, and receives the reflection signal RX using the transmitting and receiving antenna 11.

As shown in Fig. 2, the signal generating circuit 12 has an oscillator 12a and an amplifier 12b. The oscillator 12a generates a high-frequency signal having a predetermined frequency. The amplifier 12b amplifies the electric power of the high-frequency signal generated by the oscillator 12a generated to a predetermined level. Then, the output signal of the amplifier 12b is fed to the transmitting and receiving antenna 11 as the transmission signal TX. Hereinafter, the "oscillator 12a generates a high-frequency signal as the transmission signal TX and the amplifier 12b amplifies the electric power of the generated high-frequency signal" is abbreviated as "the signal generating circuit 12 generates the transmission signal TX". As the frequency of the transmission signal TX generated by the signal generating circuit 12, a frequency in a 2.4 GHz band is used.

As shown in Fig. 2, the phase shifter 13 is configured to include two transmission lines having different line lengths and two switch elements, and is disposed between the transmitting and receiving antenna 11 and the signal generating circuit 12. The two transmission lines are a first transmission line 13a and a second transmission line 13b. The two switch elements are a first switch element 13c and a second switch element 13d.

The line length of the second transmission line 13b is set so as to be larger than the line length of the first transmission line 13a by a 1/8 wavelength at the frequency of the transmission signal TX. The first switch element 13c and the second switch element 13d are interlocked with each other to perform connection switching, so that the transmitting and receiving antenna 11 and the signal generating circuit 12 are connected to each other through any one of the first transmission line 13a and the second transmission line 13b. The following explanation will be given on the assumption that a state in which the transmitting and receiving antenna 11 and the signal generating circuit 12 are connected to each other through the first transmission line 13a is referred to as a first phase state and a state in which the transmitting and receiving antenna 11 and the signal generating circuit 12 are connected to each other through the second transmission line 13b is referred to as a second phase state.

The transmission signal TX generated by the signal generating circuit 12 passes through the phase shifter 13 to be emitted to the detection target 30, and then passes through the phase shifter 13 again as the reflection signal RX. Therefore, the second phase state is a state in which the phase of the reflection signal RX is delayed by a phase corresponding to a 1/4 wavelength with respect to that in the first phase state, that is, a state in which the phase of the reflection signal RX is delayed by π/2 (radian) with respect to that in the first phase state.

As shown in Fig. 2, the first detection circuit 14 has a mixer circuit 14a, a low pass filter 14b, and a signal conversion circuit 14c. The mixer circuit 14a is connected to the signal generating circuit 12 side of the first transmission line 13a. In addition, a part of the transmission signal TX and the reflection signal RX corresponding to the first phase state are input to the mixer circuit 14a, and a mixed signal of the transmission signal TX and the reflection signal RX is output. The output signal of the mixer circuit 14a is input to the low pass filter 14b, and a low-frequency component of the mixed signal of the transmission signal TX and the reflection signal RX is output. The output signal of the low pass filter 14b is input to the signal conversion circuit 14c, and a digital signal obtained by quantizing the electric potential of the output signal of the low pass filter 14b is output.

In the present embodiment, the output signal of the signal conversion circuit 14c is a first detection signal Sa1 output from the first detection circuit 14. Since a part of the transmission signal TX and the reflection signal RX corresponding to the first phase state are input to the first detection circuit 14, the first detection signal Sa1 is a detection signal corresponding to the first phase state. In addition, the electric potential A1 of the first detection signal Sa1 is a quantized value whose magnitude and reference numeral change every moment corresponding to the movement of the detection target 30, such as the breathing or heart beat movement of the detection target 30.

As shown in Fig. 2, the second detection circuit 15 has a mixer circuit 15a, a low pass filter 15b, and a signal conversion circuit 15c. The mixer circuit 15a is connected to the signal generating circuit 12 side of the second transmission line 13b. In addition, a part of the transmission signal TX and the reflection signal RX corresponding to the second phase state are input to the mixer circuit 15a, and a mixed signal of the transmission signal TX and the reflection signal RX is output. The output signal of the mixer circuit 15a is input to the low pass filter 15b, and a low-frequency component of the mixed signal of the transmission signal TX and the reflection signal RX is output. The output signal of the low pass filter 15b is input to the signal conversion circuit 15c, and a digital signal obtained by quantizing the electric potential of the output signal of the low pass filter 15b is output.

In the present embodiment, the output signal of the signal conversion circuit 15c is a second detection signal Sa2 output from the second detection circuit 15. Since a part of the transmission signal TX and the reflection signal RX corresponding to the second phase state are input to the second detection circuit 15, the second detection signal Sa2 is a detection signal corresponding to the second phase state. In addition, the electric potential A2 of the second detection signal Sa2 is a quantized value whose magnitude and reference numeral change every moment corresponding to the movement of the detection target 30, such as the breathing or heart beat movement of the detection target 30.

The control circuit 16 controls the switching timing of the phase state of the phase shifter 13. As shown in Fig. 4, assuming that the period of the first phase state is a first period t1 and the period of the second phase state is a second period t2, the control circuit 16 controls the sensor unit 10 so that the first period t1 and the second period t2 are repeated alternately.

A switching period to that is a time obtained by adding up the first period t1 and the second period t2 is set to be sufficiently shorter than the period of a signal included in the first detection signal Sa1 or the second detection signal Sa2 so that the first detection signal Sa1 or the second detection signal Sa2 is not affected.

As shown in Figs. 1A and 1B, the signal processing unit 20 has a phase detection circuit 21 and a signal processing circuit 22. As shown in Fig. 3, the phase detection circuit 21 has two mixer circuits, two low pass filters, and an arithmetic circuit 21g. The two mixer circuits are a mixer circuit 21a and a mixer circuit 21c. The two low pass filters are a low pass filter 21b and a low pass filter 21d.

The mixer circuit 21a has two input terminals and one output terminal. The first detection signal Sa1 and the second detection signal Sa2 are input to the two input terminals of the mixer circuit 21a, and a mixed signal of the first detection signal Sa1 and the second detection signal Sa2 is output from the output terminal of the mixer circuit 21a. Since the principle of mixed signal generation using a mixer circuit is known, detailed explanation thereof will be omitted. However, in a case where two signals are input to the mixer circuit, a mixed signal proportional to the product of two input signals is output from the mixer circuit.

The low pass filter 21b levels the output signal of the mixer circuit 21a. Then, the electric potential of the output signal of the low pass filter 21b is first numerical information B1. Since the first numerical information B1 is numerical information obtained by leveling the mixed signal of the first detection signal Sa1 and the second detection signal Sa2, the first numerical information B1 is a value proportional to a value obtained by leveling the product between the electric potential A1 of the first detection signal Sa1 and the electric potential A2 of the second detection signal Sa2.

The mixer circuit 21c has two input terminals and one output terminal. The first detection signal Sa1 is input to the two input terminals of the mixer circuit 21c, and a mixed signal of the first detection signal Sa1 and the first detection signal Sa1 is output from the output terminal of the mixer circuit 21c. The low pass filter 21d levels the output signal of the mixer circuit 21c. Then, the electric potential of the output signal of the low pass filter 21d is second numerical information B2. Since the second numerical information B2 is numerical information obtained by leveling the mixed signal of the first detection signal Sa1 and the first detection signal Sa1, the square of the electric potential A1 of the first detection signal Sa1 is a value proportional to the leveled value.

The arithmetic circuit 21g is a circuit having a semiconductor for calculation. The first numerical information B1 and the second numerical information B2 are input to the arithmetic circuit 21g. Then, the arithmetic circuit 21g calculates a phase angle in the orthogonal coordinates called IQ coordinates based on the first numerical information B1 and the second numerical information B2, and transmits phase information regarding the calculated phase angle to the signal processing circuit 22. Although the IQ coordinates are orthogonal coordinates that are generally used in a digital modulation method called IQ modulation, the orthogonal coordinates used in the present embodiment are also referred to as IQ coordinates for convenience.

The signal processing circuit 22 is a circuit having a semiconductor for signal processing. The first detection signal Sa1 and the second detection signal Sa2 are input to the signal processing circuit 22. In addition, the phase information described above is transmitted to the signal processing circuit 22 from the phase detection circuit 21. Then, the signal processing circuit 22 generates a signal before correction Sc for movement analysis based on the first detection signal Sa1 and the second detection signal Sa2, and generates a correction signal Sd by correcting the signal before correction Sc based on the transmitted phase information. Then, the signal processing circuit 22 analyzes the movement of the detection target 30 using the correction signal Sd, and outputs movement information Sout corresponding to the analysis result.

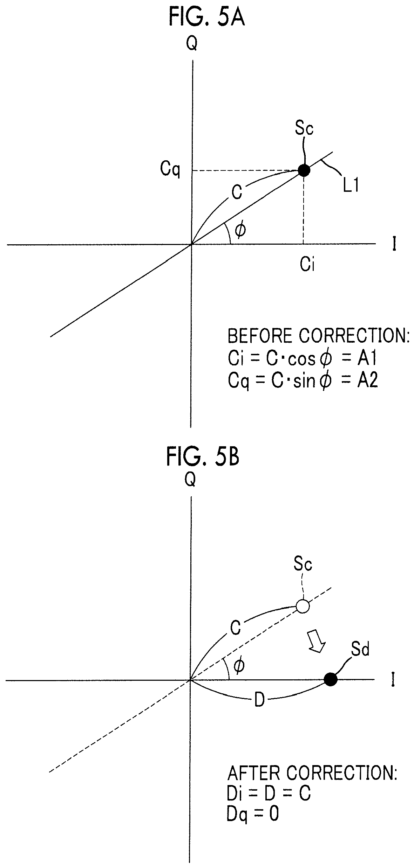

Next, a method of generating the signal before correction Sc for movement analysis and a method of correcting the signal before correction Sc for movement analysis will be described with reference to Figs. 5A and 5B. Figs. 5A and 5B are explanatory diagrams regarding a phase correction method according to the first embodiment of the present invention. Fig. 5A is an explanatory diagram showing the state of the signal before correction Sc, and Fig. 5B is an explanatory diagram showing the state of the correction signal Sd. In Figs. 5A and 5B, the horizontal axis indicates an I coordinate axis in the IQ coordinates, and the vertical axis indicates a Q coordinate axis in the IQ coordinates.

First, the method of generating the signal before correction Sc will be described. As described above, the first detection signal Sa1 is a signal corresponding to the first phase state, and the second detection signal Sa2 is a signal corresponding to the second phase state in which the phase of a reflection signal is delayed by π/2 (radian) with respect to that in the first phase state. Therefore, the first detection signal Sa1 and the second detection signal Sa2 can be used as an I coordinate component (cosine component) and a Q coordinate component (sine component) in the orthogonal coordinates called IQ coordinates. In the present embodiment, the signal before correction Sc is generated so that the electric potential A1 of the first detection signal Sa1 becomes the I coordinate component of the signal before correction Sc and the electric potential A2 of the second detection signal Sa2 becomes the Q coordinate component of the signal before correction Sc.

The signal before correction Sc generated as described above is a signal whose amplitude and phase change every moment corresponding to the movement of the detection target 30, such as the breathing or heart beat movement of the detection target 30. Then, in a case where there is no disturbance noise or there is no movement of the detection target 30, the signal before correction Sc is changed along a line L1 on the IQ coordinates in which an angle with respect to the I coordinate axis passing through the origin is φ, as shown in Fig. 5A. The following explanation will be given on the assumption that the I coordinate component of the signal before correction Sc at a certain moment is an instantaneous I coordinate component Ci, the Q coordinate component of the signal before correction Sc at the certain moment is an instantaneous Q coordinate component Cq, and the amplitude (distance from the origin) of the signal before correction Sc at the certain moment is an instantaneous amplitude C. In addition, the explanation will be given on the assumption that the angle φ of the line L1 with respect to the I coordinate axis is the phase angle φ of the signal before correction Sc.

In a case where the signal before correction Sc is the signal described above, the relationship of Ci = C·cosφ = A1 is satisfied among the instantaneous I coordinate component Ci, the instantaneous amplitude C, the phase angle φ, and the electric potential A1 . In addition, the relationship of Cq = C·sinφ = A2 is satisfied among the instantaneous Q coordinate component Cq, the instantaneous amplitude C, the phase angle φ, and the electric potential A2. Thus, the electric potential A1 of the first detection signal Sa1 is a value proportional to the cosine function for the phase angle φ, and the electric potential A2 of the second detection signal Sa2 is a value proportional to the sine function for the phase angle φ.

The phase angle φ is an angle determined by the distance between the wireless sensor device 1 and the detection target 30. In a case where the distance between the wireless sensor device 1 and the detection target 30 is changed, the phase angle φ is also changed according to the change in the distance between the wireless sensor device 1 and the detection target 30.

Next, a method of calculating the phase angle φ will be described. As described above, the first numerical information B1 that is an output signal of the low pass filter 21b is a value proportional to a value obtained by leveling the product ((C·cosφ)·(C·sinφ)) between the electric potential A1 of the first detection signal Sa1 and the electric potential A2 of the second detection signal Sa2, that is, a value proportional to the product between the cosine function and the sine function for the phase angle φ. In addition, the second numerical information B2 that is an output signal of the low pass filter 21d is a value proportional to a value obtained by leveling the square ((C·cosφ) ^2) of the electric potential A1 of the first detection signal Sa1, that is, a value proportional to the square of the cosine function for the phase angle φ.

Thus, the first numerical information B1 is a value proportional to the product of the cosine function and the sine function for the phase angle φ, and the second numerical information B2 is a value proportional to the square of the cosine function for the phase angle φ. Therefore, the relationship of B1/B2 = sinφ/cosφ = tanφ is satisfied among the first numerical information B1, the second numerical information B2, and the phase angle φ. In addition, using the relationship, the phase angle φ can be calculated as an arc tangent function (tan-1 (B1/B2)) of a value obtained by dividing the first numerical information B1 by the second numerical information B2.

Using such a relationship, the phase detection circuit 21 calculates the phase angle φ using the first numerical information B1 and the second numerical information B2, and outputs the phase angle φ to the signal processing circuit 22 as phase information.

Next, a method of correcting the signal before correction Sc will be described. The signal processing circuit 22 generates the correction signal Sd by generating the signal before correction Sc and then correcting the signal before correction Sc based on the phase angle φ. In the present embodiment, the correction based on the phase information is to shift the phase of the signal before correction Sc by -φ obtained by inverting the sign of the phase angle φ, as shown in Fig. 5B. That is, this is equivalent to converting the signal before correction Sc to a signal whose instantaneous amplitude is the same as the signal before correction Sc and phase angle is 0°.

Assuming that the I coordinate component of the correction signal Sd at a certain moment is an instantaneous I coordinate component Di, the Q coordinate component of the correction signal Sd at the certain moment is an instantaneous Q coordinate component Dq, and the amplitude (distance from the origin) of the correction signal Sd at the certain moment is an instantaneous amplitude D, the instantaneous I coordinate component Di of the correction signal Sd is a value equal to the instantaneous amplitude D of the correction signal Sd, that is, a value equal to the instantaneous amplitude C of the signal before correction Sc. The instantaneous Q coordinate component Dq of the correction signal Sd is 0. In addition, the correction signal Sd is a signal that changes along the I coordinate axis according to the change in the signal before correction Sc.

Thus, the correction signal Sd whose phase angle has been converted into 0° is a signal that is hardly influenced by changes in the phase angle φ even if the phase angle φ is changed by a change in the distance between the wireless sensor device 1 and the detection target 30. Then, the signal processing circuit 22 analyzes the movement of the detection target 30 based on the temporal change of the correction signal Sd, and outputs the movement information Sout corresponding to the analysis result. Since the method of analyzing the movement of the detection target 30 using the correction signal Sd is known, the detailed explanation thereof will be omitted.

Next, the effect of the present embodiment will be described. In a device such as the wireless sensor device 1 of the present embodiment, if the distance between the wireless sensor device 1 and the detection target 30 is changed due to the movement of the detection target 30 while the movement of the detection target 30 is being detected, the phase difference between the transmission signal TX and the reflection signal RX is changed corresponding to the distance change. In addition, in such a device, a detection signal for detecting the movement of the detection target 30 is generated using the phase difference between the transmission signal TX and the reflection signal RX. For this reason, when the phase difference between the transmission signal TX and the reflection signal RX is under specific conditions called a null point, detection sensitivity when detecting the movement of the detection target 30 is greatly reduced. Accordingly, there have been cases where it is difficult to detect the movement of the detection target 30.

Since the principle of generating a null point is known, the detailed explanation thereof will be omitted. The detection sensitivity is greatly reduced when the phase difference between the transmission signal TX and the reflection signal RX is 0 or π (radian), and the detection sensitivity is good when the phase difference between the transmission signal TX and the reflection signal RX is an intermediate state, that is, when the phase difference between the transmission signal TX and the reflection signal RX is around π/2 (radian) or -π/2 (radian).

Even in a case where the detection sensitivity is not reduced greatly, if the distance between the wireless sensor device 1 and the detection target 30 is frequently changed and the phase difference between the transmission signal TX and the reflection signal RX is changed corresponding to the distance change, the amplitude or phase of a signal for analyzing the movement of the detection target 30 is also changed due to the influence. For this reason, there has been a possibility that the analysis of the movement of the detection target 30 will become complicated. Also in this case, the detection sensitivity when detecting the movement of the detection target 30 is reduced.

In contrast, in the wireless sensor device 1 of the present embodiment, the sensor unit 10 generates the first detection signal Sa1 corresponding to the first phase state, which is the phase state of the reflection signal RX, and the second detection signal Sa2 corresponding to the second phase state in which the phase of the reflection signal RX is different from that in the first phase state. Therefore, even if the distance between the wireless sensor device 1 and the detection target 30 is changed to reduce the detection sensitivity in one of the first and second phase states, it is possible to create a state with good detection sensitivity by performing switching to the other phase state.

For example, in a case where the phase difference between the transmission signal TX and the reflection signal RX in the first phase state is 0 (null point state), the detection sensitivity of the first detection signal Sa1 corresponding to the first phase state is greatly reduced. However, since the phase difference between the transmission signal TX and the reflection signal RX in the second phase state is -π/2 (radian), the detection sensitivity of the second detection signal Sa2 corresponding to the second phase state is good.

In addition, since the first detection signal Sa1 and the second detection signal Sa2 are signals corresponding to two different phase states, the first detection signal Sa1 and the second detection signal Sa2 are signals suitable for detecting phase information. The signal processing unit 20 detects phase information based on the first detection signal Sa1 and the second detection signal Sa2, and performs predetermined signal processing based on the detected phase information. Therefore, even in a case where the distance between the wireless sensor device 1 and the detection target 30 is frequently changed and the phase difference between the transmission signal TX and the reflection signal RX is changed, it is possible to detect the phase information based on the first detection signal Sa1 and the second detection signal Sa2 and correct the signal based on the detected phase information. As a result, it is possible to suppress a reduction in detection sensitivity according to a change in the distance between the wireless sensor device 1 and the detection target 30.

In the wireless sensor device 1 of the present embodiment, the second phase state is a phase state in which the phase of the reflection signal RX is different from that in the first phase state by π/2 (radian) . Therefore, by setting the first detection signal Sa1 and the second detection signal Sa2 corresponding to such two phase states as two coordinate components in the orthogonal coordinates called IQ coordinates and using the first numerical information B1 and the second numerical information B2 based on the first detection signal Sa1 and the second detection signal Sa2, it is possible to easily calculate the phase angle φ in the IQ coordinates. In addition, the signal processing unit 20 calculates the phase angle φ in the IQ coordinates using the first numerical information B1 and the second numerical information B2 based on the first detection signal Sa1 and the second detection signal Sa2, and corrects the phase of the signal before correction Sc based on the calculated phase angle φ. Then, by correcting the phase of the signal before correction Sc, the signal before correction Sc can be converted into the correction signal Sd that is hardly influenced by changes in the phase angle φ. As a result, since the influence of the change of the signal due to the change in the phase difference between the transmission signal TX and the reflection signal RX can be further reduced, it is possible to further suppress a reduction in detection sensitivity.

In the wireless sensor device 1 of the present embodiment, the second phase state is a phase state in which the phase of the reflection signal is delayed by π/2 (radian) with respect to that in the first phase state. Accordingly, the first detection signal Sa1 corresponding to the first phase state can be used as an I coordinate component (cosine component) in the IQ coordinates, and the second detection signal Sa2 corresponding to the second phase state can be used as a Q coordinate component (sine component) in the IQ coordinates.

In addition, the first numerical information B1 is numerical information obtained by leveling the mixed signal of the first detection signal Sa1 and the second detection signal Sa2. Accordingly, the first numerical information B1 is a value proportional to a value obtained by leveling the product ((C·cosφ)-(C·sinφ)) between the electric potential A1 of the first detection signal Sa1 and the electric potential A2 of the second detection signal Sa2, that is, a value proportional to the product between the cosine function and the sine function for the phase angle φ. In addition, the second numerical information B2 is numerical information obtained by leveling the mixed signal of the first detection signal Sa1 and the first detection signal Sa1. Accordingly, the second numerical information B2 is a value proportional to a value obtained by leveling the square ((C·cosφ)^2) of the electric potential A1 of the first detection signal Sa1, that is, a value proportional to the square of the cosine function for the phase angle φ. Therefore, the phase angle φ can be calculated using a simple calculation expression of an arc tangent function (tan-1(B1/B2)) of a value obtained by dividing the first numerical information B1 by the second numerical information B2. As a result, it becomes easy to calculate the phase angle φ.

In the present embodiment, the phase angle φ is calculated using the first numerical information B1 and the second numerical information B2 that are leveled by the low pass filter 21b and the low pass filter 21d, respectively, and the signal before correction Sc is corrected based on the calculated phase angle φ. In a case where a noise source is present around the wireless sensor device 1, it is possible to reduce the influence of external noise by calculating the phase angle φ using the first numerical information B1 and the second numerical information B2 that are leveled by the low pass filter 21b and the low pass filter 21d.

In the wireless sensor device 1 of the present embodiment, the sensor unit 10 can easily generate the first detection signal Sa1 corresponding to the first phase state and the second detection signal Sa2 corresponding to the second phase state using the phase shifter 13, the first detection circuit 14, and the second detection circuit 15. In addition, the signal processing unit 20 can easily perform the detection of phase information, such as the phase angle φ, and signal processing, such as phase angle correction, using the phase detection circuit 21 and the signal processing circuit 22 . As a result, in the wireless sensor device 1 of the present embodiment, it is possible to easily detect the movement of the detection target 30.

### [Second embodiment]

Hereinafter, a second embodiment of the present invention will be described with reference to the diagrams. In the present embodiment, the same components as in the first embodiment described above are denoted by the same reference numerals, and the detailed explanation thereof will be omitted.

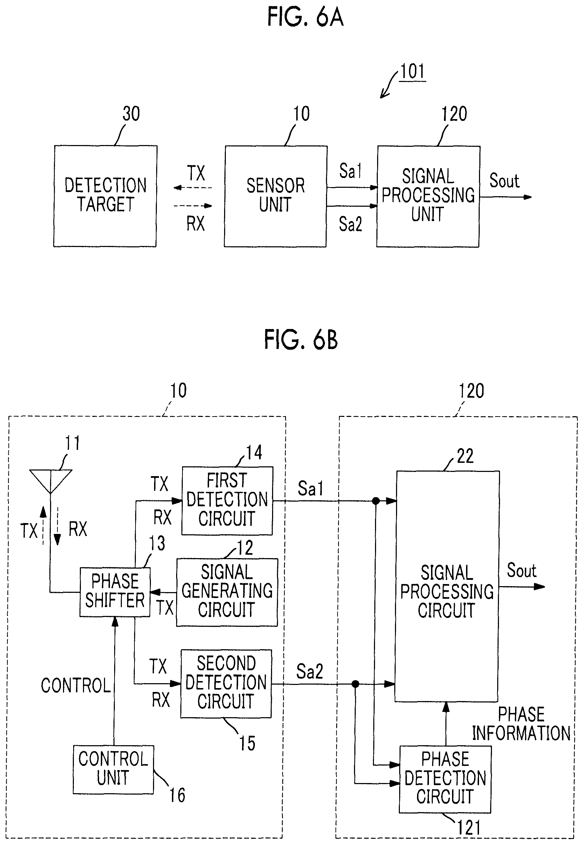

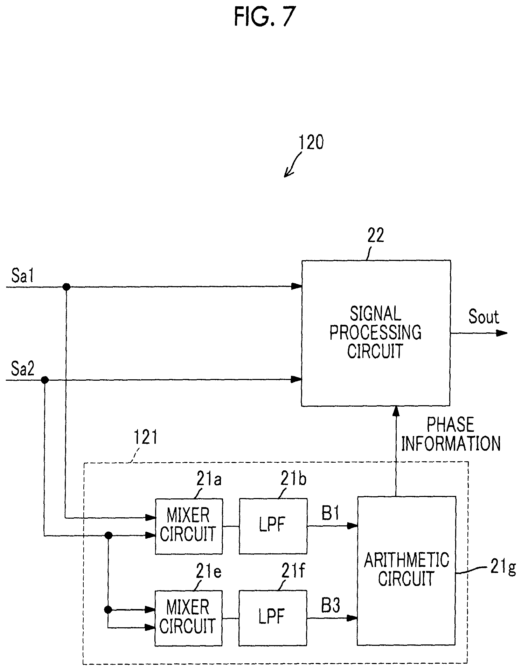

First, the configuration of a wireless sensor device 101 according to the second embodiment of the present invention will be described with reference to Figs. 6A, 6B, and 7. Figs. 6A and 6B are explanatory diagrams showing the configuration of the wireless sensor device 101 according to the second embodiment of the present invention. Fig. 7 is an explanatory diagram showing the configuration of a signal processing unit 120 shown in Figs. 6A and 6B in more detail.

As shown in Figs. 6A and 6B, the wireless sensor device 101 includes a sensor unit 10 and the signal processing unit 120. As shown in Figs. 6A and 6B, the signal processing unit 120 has a phase detection circuit 121 and a signal processing circuit 22. Thus, in the wireless sensor device 101, the signal processing unit 20 of the wireless sensor device 1 according to the first embodiment is replaced with the signal processing unit 120. In addition, in the signal processing unit 120, the phase detection circuit 21 of the signal processing unit 20 is replaced with the phase detection circuit 121.

As shown in Fig. 7, the phase detection circuit 121 has two mixer circuits, two low pass filters, and an arithmetic circuit 21g. The two mixer circuits are a mixer circuit 21a and a mixer circuit 21e . The two low pass filters are a low pass filter 21b and a low pass filter 21f. Thus, in the phase detection circuit 121, the mixer circuit 21c and the low pass filter 21d of the phase detection circuit 21 are replaced with the mixer circuit 21e and the low pass filter 21f.

The mixer circuit 21e has two input terminals and one output terminal. The second detection signal Sa2 is input to the two input terminals of the mixer circuit 21e, and a mixed signal of the second detection signal Sa2 and the second detection signal Sa2 is output from the output terminal of the mixer circuit 21e. The low pass filter 21f levels the output signal of the mixer circuit 21e. Then, the electric potential of the output signal of the low pass filter 21f is third numerical information B3. Since the third numerical information B3 is numerical information obtained by leveling the mixed signal of the second detection signal Sa2 and the second detection signal Sa2, the square ((C·sinφ)^2) of the electric potential A2 of the second detection signal Sa2 is a value proportional to the leveled value.

The configuration of the arithmetic circuit 21g is the same as that in the first embodiment, but the first numerical information B1 and the third numerical information B3 are input to the arithmetic circuit 21g. Then, the arithmetic circuit 21g calculates the phase angle φ in the orthogonal coordinates called IQ coordinates using the first numerical information B1 and the third numerical information B3.

As in the first embodiment, the first numerical information B1 is a value proportional to a value obtained by leveling the product ((C·cosφ)-(C·sinφ)) between the electric potential A1 of the first detection signal Sa1 and the electric potential A2 of the second detection signal Sa2, that is, a value proportional to the product between the cosine function and the sine function for the phase angle φ. On the other hand, as described above, the third numerical information B3 is a value proportional to a value obtained by leveling the square ((C·sinφ)^2) of the electric potential A2 of the second detection signal Sa2, that is, a value proportional to the square of the sine function for the phase angle φ.

Thus, the first numerical information B1 is a value proportional to the product of the cosine function and the sine function for the phase angle φ, and the third numerical information B3 is a value proportional to the square of the sine function for the phase angle φ. Therefore, the relationship of B3/B1 = sinφ/cosφ = tanφ is satisfied among the first numerical information B1, the third numerical information B3, and the phase angle φ. In addition, using the relationship, the phase angle φ can be calculated as an arc tangent function (tan-1(B3/B1)) of a value obtained by dividing the third numerical information B3 by the first numerical information B1.

In the present embodiment, the phase detection circuit 121 calculates the phase angle φ as an arc tangent function (tan-1(B3/B1)) of a value, which is obtained by dividing the third numerical information B3 by the first numerical information B1, using the relationship, and outputs the phase angle φ to the signal processing circuit 22 as phase information.

As in the first embodiment, the signal processing circuit 22 generates the signal before correction Sc based on the first detection signal Sa1 and the second detection signal Sa2, and generates the correction signal Sd by correcting the signal before correction Sc based on the phase angle φ. Then, the signal processing circuit 22 analyzes the movement of the detection target 30 using the correction signal Sd, and outputs the movement information Sout corresponding to the analysis result.

Next, the effect of the present embodiment will be described. In the present embodiment, only the effect different from that of the first embodiment will be described. In the wireless sensor device 101 of the present embodiment, the second phase state is a phase state in which the phase of the reflection signal is delayed by π/2 (radian) with respect to that in the first phase state. Accordingly, the first detection signal Sa1 corresponding to the first phase state can be used as an I coordinate component (cosine component) in the IQ coordinates, and the second detection signal Sa2 corresponding to the second phase state can be used as a Q coordinate component (sine component) in the IQ coordinates.

In addition, the first numerical information B1 is numerical information obtained by leveling the mixed signal of the first detection signal Sa1 and the second detection signal Sa2. Accordingly, the first numerical information B1 is a value proportional to a value obtained by leveling the product ((C·cosφ)·(C·sinφ)) between the electric potential A1 of the first detection signal Sa1 and the electric potential A2 of the second detection signal Sa2, that is, a value proportional to the product between the cosine function and the sine function for the phase angle φ. In addition, the third numerical information B3 is numerical information obtained by leveling the mixed signal of the second detection signal Sa2 and the second detection signal Sa2. Accordingly, the third numerical information B3 is a value proportional to a value obtained by leveling the square ((C·sinφ)^2) of the electric potential A2 of the second detection signal Sa2, that is, a value proportional to the square of the sine function for the phase angle φ. Therefore, the phase angle φ can be calculated using a simple calculation expression of an arc tangent function (tan-1(B3/B1)) of a value obtained by dividing the third numerical information B3 by the first numerical information B1. As a result, as in the first embodiment, it becomes easy to calculate the phase angle φ.

### [Third embodiment]

Hereinafter, a third embodiment of the present invention will be described with reference to the diagrams . In the present embodiment, the same components as in the first or second embodiment described above are denoted by the same reference numerals, and the detailed explanation thereof will be omitted.

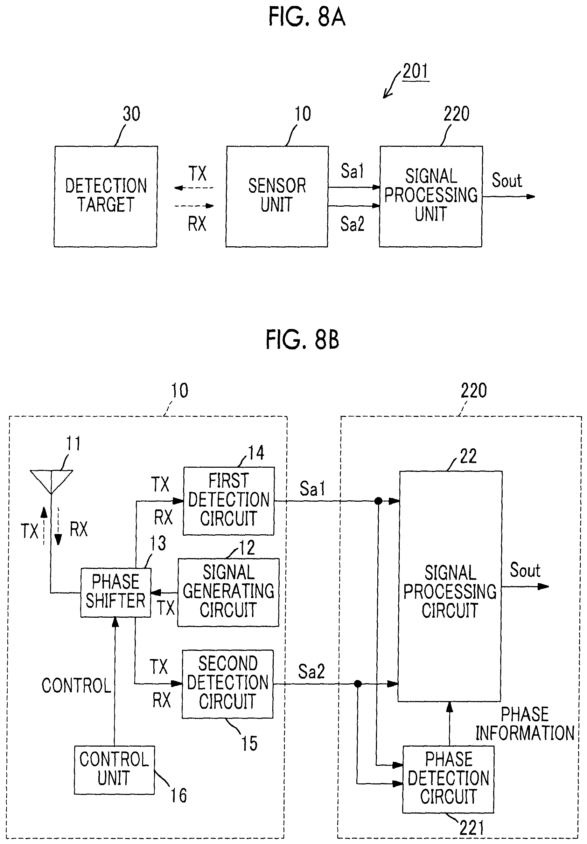

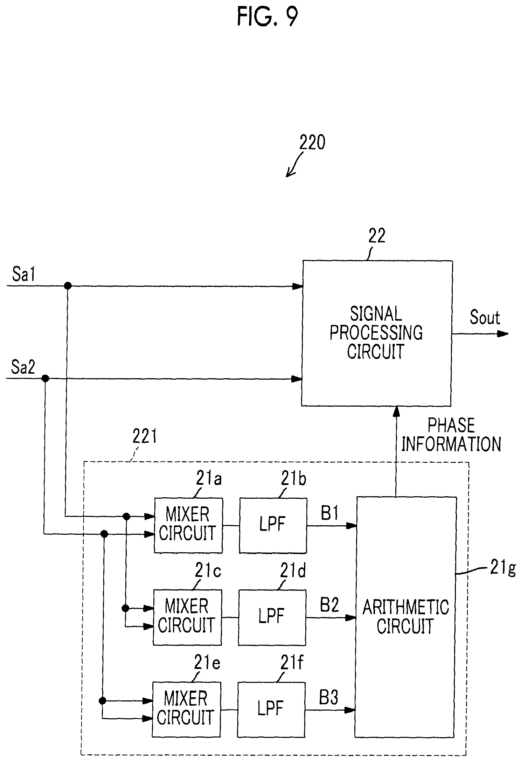

First, the configuration of a wireless sensor device 201 according to the third embodiment of the present invention will be described with reference to Figs. 8A, 8B, and 9. Figs. 8A and 8B are explanatory diagrams showing the configuration of the wireless sensor device 201 according to the third embodiment of the present invention. Fig. 9 is an explanatory diagram showing the configuration of a signal processing unit 220 shown in Figs. 8A and 8B in more detail.

As shown in Figs. 8A and 8B, the wireless sensor device 201 includes a sensor unit 10, the signal processing unit 220, and a control circuit 16. As shown in Figs. 8A and 8B, the signal processing unit 220 has a phase detection circuit 221 and a signal processing circuit 22. Thus, in the wireless sensor device 201, the signal processing unit 20 of the wireless sensor device 1 according to the first embodiment is replaced with the signal processing unit 220. In addition, in the signal processing unit 220, the phase detection circuit 21 of the signal processing unit 20 is replaced with the phase detection circuit 221.

As shown in Fig. 9, the phase detection circuit 221 has three mixer circuits, three low pass filters, and an arithmetic circuit 21g. The three mixer circuits are a mixer circuit 21a, a mixer circuit 21c, and a mixer circuit 21e. The three low pass filters are a low pass filter 21b, a low pass filter 21d, and a low pass filter 21f. Thus, the phase detection circuit 221 is formed by adding the mixer circuit 21e and the low pass filter 21f to the phase detection circuit 21.

The mixer circuit 21e has two input terminals and one output terminal. The second detection signal Sa2 is input to the two input terminals of the mixer circuit 21e, and a mixed signal of the second detection signal Sa2 and the second detection signal Sa2 is output from the output terminal of the mixer circuit 21e. The low pass filter 21f levels the output signal of the mixer circuit 21e. Then, the electric potential of the output signal of the low pass filter 21f is third numerical information B3. Since the third numerical information B3 is numerical information obtained by leveling the mixed signal of the second detection signal Sa2 and the second detection signal Sa2, the square ((C·sinφ)^2) of the electric potential A2 of the second detection signal Sa2 is a value proportional to the leveled value.

The configuration of the arithmetic circuit 21g is the same as that in the first embodiment, the first numerical information B1, the second numerical information B2, and the third numerical information B3 are input to the arithmetic circuit 21g. Then, the arithmetic circuit 21g calculates the phase angle φ in the orthogonal coordinates called IQ coordinates using the first numerical information B1, the second numerical information B2, and the third numerical information B3.

As in the first embodiment, the first numerical information B1 is a value proportional to a value obtained by leveling the product ((C·cosφ)-(C·sinφ)) between the electric potential A1 of the first detection signal Sa1 and the electric potential A2 of the second detection signal Sa2, that is, a value proportional to the product between the cosine function and the sine function for the phase angle φ. In addition, as in the first embodiment, the second numerical information B2 is a value proportional to a value obtained by leveling the square ((C·cosφ)^2) of the electric potential A1 of the first detection signal Sa1, that is, a value proportional to the square of the cosine function for the phase angle φ. In addition, as in the second embodiment, the third numerical information B3 is a value proportional to a value obtained by leveling the square ((C·sinφ)^2) of the electric potential A2 of the second detection signal Sa2, that is, a value proportional to the square of the sine function for the phase angle φ.

Thus, the first numerical information B1 is a value proportional to the product of the cosine function and the sine function for the phase angle φ, the second numerical information B2 is a value proportional to the square of the cosine function for the phase angle φ, and the third numerical information B3 is a value proportional to the square of the sine function for the phase angle φ. Therefore, two relationships of B1/B2 = sinφ/cosφ = tanφ and B3/B1 = sinφ/cosφ = tanφ are satisfied among the first numerical information B1, the second numerical information B2, and the third numerical information B3.

In the present embodiment, using the relationships, the phase detection circuit 221 calculates a first calculation value of the phase angle φ as an arc tangent function (tan-1(B1/B2)) for a value obtained by dividing the first numerical information B1 by the second numerical information B2, and calculates a second calculation value of the phase angle φ as an arc tangent function (tan-1(B3/B1)) for a value obtained by dividing the third numerical information B3 by the first numerical information B1. Then, the phase detection circuit 221 outputs the first and second calculation values to the signal processing circuit 22 as phase information.

As in the first embodiment, the signal processing circuit 22 generates the signal before correction Sc based on the first detection signal Sa1 and the second detection signal Sa2, and generates the correction signal Sd by correcting the phase of the signal before correction Sc based on the phase angle φ. Then, the signal processing circuit 22 analyzes the movement of the detection target 30 using the correction signal Sd, and outputs the movement information Sout corresponding to the analysis result. In the present embodiment, however, the signal processing circuit 22 selects one of the first and second calculation values of the phase angle φ according to a predetermined standard, and corrects the phase of the signal before correction Sc using the selected calculation value.

There are various methods of selecting one of the first and second calculation values as the phase angle φ. In the present embodiment, the signal processing circuit 22 selects the phase angle φ based on the magnitude (absolute value) of the electric potential A1 and the magnitude (absolute value) of the electric potential A2.

For example, in a case where the magnitude (absolute value) of the electric potential A1 is larger than the magnitude (absolute value) of the electric potential A2, the calculation accuracy of the phase angle φ when calculating the phase angle φ using the second numerical information B2 proportional to the square of A1 is likely to be higher than that when calculating the phase angle φ using the third numerical information B3 proportional to the square of A2. In such a case, therefore, the first calculation value is selected as the phase angle φ. In addition, in a case where the magnitude (absolute value) of the electric potential A2 is larger than the magnitude (absolute value) of the electric potential A1, the calculation accuracy of the phase angle φ when calculating the phase angle φ using the third numerical information B3 proportional to the square of A2 is likely to be higher than that when calculating the phase angle φ using the second numerical information B2 proportional to the square of A1. In such a case, therefore, the second calculation value is selected as the phase angle φ.

Next, the effect of the present embodiment will be described. In the present embodiment, only the effect different from that of the first embodiment will be described. In the wireless sensor device 201 of the present embodiment, the second phase state is a phase state in which the phase of the reflection signal is delayed by π/2 (radian) with respect to that in the first phase state. Accordingly, the first detection signal Sa1 corresponding to the first phase state can be used as an I coordinate component (cosine component) in the IQ coordinates, and the second detection signal Sa2 corresponding to the second phase state can be used as a Q coordinate component (sine component) in the IQ coordinates.

In addition, the phase angle φ can be calculated using two calculation methods of a calculation method for calculating the phase angle φ as the arc tangent function (tan-1(B1/B2)) of the value obtained by dividing the first numerical information B1 by the second numerical information B2 and a calculation method for calculating the phase angle φ as the arc tangent function (tan-1(B3/B1)) of the value obtained by dividing the third numerical information B3 by the first numerical information B1. As a result, since it is possible to calculate the phase angle φ using simple mathematical expressions, it becomes easy to calculate the phase angle φ as in the first or second embodiment.

In addition, in the wireless sensor device 201 of the present embodiment, the first calculation value of the phase angle φ is calculated as the arc tangent function (tan-1(B1/B2)) for the value obtained by dividing the first numerical information B1 by the second numerical information B2, and the second calculation value of the phase angle φ is calculated as the arc tangent function (tan-1(B3/B1)) for the value obtained by dividing the third numerical information B3 by the first numerical information B1. Then, one of the first and second calculation values is selected as a calculation value of the phase angle φ. Therefore, even in a case where the calculation accuracy of one of the first and second calculation values is reduced, it is possible to suppress a reduction in detection sensitivity by using the other one as a calculation value of the phase angle φ.

### [Fourth embodiment]

Hereinafter, a fourth embodiment of the present invention will be described with reference to the diagrams. In the present embodiment, the same components as in the first embodiment described above are denoted by the same reference numerals, and the detailed explanation thereof will be omitted.

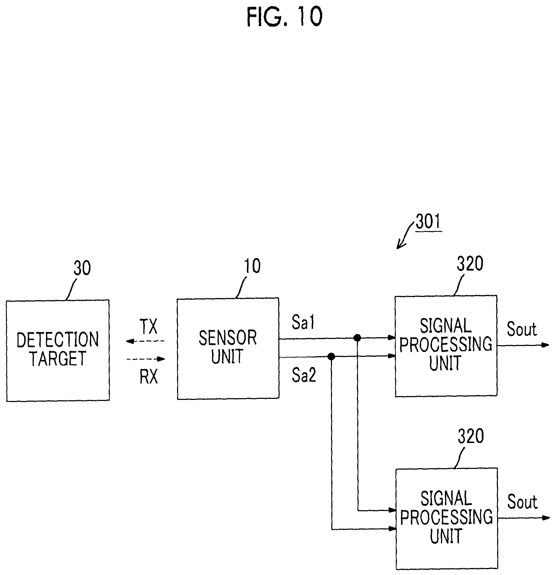

First, the configuration of a wireless sensor device 301 according to the fourth embodiment of the present invention will be described with reference to Fig. 10. Fig. 10 is an explanatory diagram showing the configuration of the wireless sensor device 301 according to the fourth embodiment of the present invention.

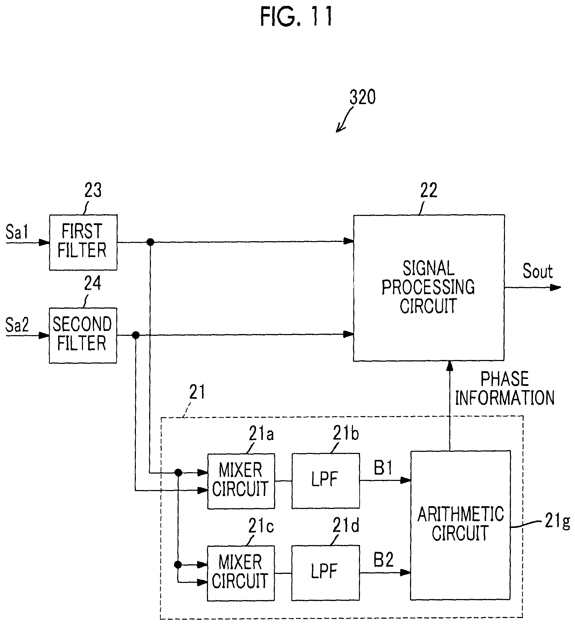

As shown in Fig. 10, the wireless sensor device 301 includes a sensor unit 10 and two signal processing units 320. As shown in Fig. 11, the signal processing unit 320 has a phase detection circuit 21, a signal processing circuit 22, a first filter 23, and a second filter 24. Thus, the wireless sensor device 301 is a wireless sensor device having a plurality of signal processing units. The signal processing unit 320 has a configuration obtained by adding the first filter 23 and the second filter 24 to the signal processing unit 20 of the first embodiment.

The first filter 23 is a band pass filter having a predetermined pass band at a low frequency. A first detection signal Sa1 is input to the first filter 23, and a predetermined frequency component suitable for the movement of the detection target 30, which needs to be detected, is extracted from the first detection signal Sa1. Then, in the present embodiment, instead of the first detection signal Sa1, the output signal of the first filter 23 is input to the phase detection circuit 21 and the signal processing circuit 22.

The second filter 24 is a band pass filter having the same pass band as the first filter 23. A second detection signal Sa2 is input to the second filter 24, and a predetermined frequency component suitable for the movement of the detection target 30, which needs to be detected, is extracted from the second detection signal Sa2. Then, in the present embodiment, instead of the second detection signal Sa2, the output signal of the second filter 24 is input to the phase detection circuit 21 and the signal processing circuit 22.

The pass band of the first filter 23 and the pass band of the second filter 24 are different bands for each signal processing unit 320. That is, in one of the two signal processing units 320, the pass band of the first filter 23 and the pass band of the second filter 24 are pass bands (about 0.7 Hz to 1 Hz) corresponding to the breathing of a person. In the other one of the two signal processing units 320, the pass band of the first filter 23 and the pass band of the second filter 24 are pass bands (about 1 Hz to 2 Hz) corresponding to the heart beat movement of a person.

Then, in the present embodiment, one of the two signal processing units 320 performs movement analysis regarding the breathing of a person, and outputs the movement information regarding the breathing of the person. In addition, the other one of the two signal processing units 320 performs movement analysis regarding the heart beat movement of a person, and outputs the movement information regarding the heart beat movement of the person.

Next, the effect of the present embodiment will be described. In the present embodiment, only the effect different from that of the first embodiment will be described. In the wireless sensor device 301 of the present embodiment, it is possible to detect a plurality of different pieces of movement information from the movement of the detection target 30 using a plurality of signal processing units 320. In the case of detecting a plurality of pieces of movement information, there is a case in which frequencies suitable for detection are different for each of the pieces of movement information. In the wireless sensor device 301 of the present embodiment, however, the pass band of the first filter 23 and the pass band of the second filter 24 are different bands for each signal processing unit 320. Therefore, it is possible to extract a frequency component suitable for the detection of movement for each signal processing unit 320 from the detection signal output from the sensor unit 10. As a result, in the wireless sensor device 301 of the present embodiment, it is possible to efficiently detect a plurality of different movements of the detection target 30.

While the embodiments of the present invention have been described above, the present invention is not limited to the above-described embodiments, and can be appropriately modified without departing from the scope of the object of the present invention.

For example, the movement detected in the embodiments of the present invention may be a movement other than that described above. For example, the movement to be detected may be a movement other than the heart beat movement or breathing such as pulsation of a person. In addition, the movement to be detected may be the periodic movement of an object other than a person.

In the embodiments of the present invention, the transmitting and receiving antenna 11 may be an antenna obtained by combining a transmission antenna and a receiving antenna. A circuit, such as a filter or an amplifier, may be connected to each of the transmission antenna and the receiving antenna.

In the embodiments of the present invention, the frequency of the transmission signal TX generated by the signal generating circuit 12 may be a frequency other than the frequency described above. For example, the frequency of the transmission signal TX may be a frequency in a band of hundreds of MHz or a 5 GHz band.

In the embodiments of the present invention, the configuration of the phase shifter 13 may be a configuration other than that described above if switching to two phase states is possible. For example, the phase shifter 13 may have a configuration in which a variable capacitance element or the like is connected to the end of a transmission line having a predetermined line length.

In the embodiments of the present invention, the transmission signals TX of two different phase states may be input to the first detection circuit 14 and the second detection circuit 15. In this case, the reflection signals RX having the same phase state may be input to the first detection circuit 14 and the second detection circuit 15. A reduction in detection sensitivity occurs due to the phase difference between the transmission signal TX and the reflection signal RX. Therefore, even in a case where the transmission signals TX of two different phase states are used as described above, it is possible to obtain the same effect as in the case of using the reflection signals RX of two different phase states.

In the embodiments of the present invention, the first detection circuit 14 of the sensor unit 10 may not have the signal conversion circuit 14c, and the second detection circuit 15 may not have the signal conversion circuit 15c. In this case, the first detection signal Sa1 and the second detection signal Sa2 are analog detection signals. However, even if the first detection signal Sa1 and the second detection signal Sa2 are analog detection signals, it is possible to obtain the same effect.

In the third embodiment of the present invention, the phase detection circuit 221 may determine which of the phase angles φ calculated by two calculation methods is to be transmitted to the signal processing circuit 22, and the phase detection circuit 221 may transmit only the phase information regarding one of the phase angles φ calculated by two calculation methods to the signal processing circuit 22.

In the third embodiment of the present invention, the signal processing circuit 22 selects one of the first and second calculation values of the phase angle φ, and uses the selected calculation value as a calculation value of the phase angle φ. However, the signal processing circuit 22 may use an average value of the first and second calculation values as a calculation value of the phase angle φ. Even in such a case, when the calculation accuracy of one of the first and second calculation values is reduced, it is possible to suppress a reduction in detection sensitivity by reducing the influence of the calculation value on the side where the calculation accuracy has been reduced.

In the fourth embodiment of the present invention, the number of signal processing units 20 provided in the wireless sensor device 301 may be 3 or more. In addition, the signal processing unit provided in the wireless sensor device 301 may not be the signal processing unit 20 shown in the first embodiment, but may be the signal processing unit 120 shown in the second embodiment or the signal processing unit 220 shown in the third embodiment.

- 1:: wireless sensor device
- 10:: sensor unit
- 11:: transmitting and receiving antenna
- 12:: signal generating circuit
- 12a:: oscillator
- 12b:: amplifier
- 13:: phase shifter
- 13a:: first transmission line
- 13b:: second transmission line
- 13c:: first switch element
- 13d:: second switch element
- 14:: first detection circuit
- 14a:: mixer circuit
- 14b:: low pass filter
- 14c:: signal conversion circuit
- 15:: second detection circuit
- 15a:: mixer circuit
- 15b:: low pass filter
- 15c:: signal conversion circuit
- 16:: control circuit
- 20:: signal processing unit
- 21:: phase detection circuit
- 21a:: mixer circuit
- 21b:: low pass filter
- 21c:: mixer circuit
- 21d:: low pass filter
- 21e:: mixer circuit
- 21f:: low pass filter
- 21g:: arithmetic circuit
- 22:: signal processing circuit
- 23:: first filter
- 24:: second filter
- 30:: detection target
- 101:: wireless sensor device
- 120:: signal processing unit
- 121:: phase detection circuit
- 201:: wireless sensor device
- 220:: signal processing unit
- 221:: phase detection circuit
- 301:: wireless sensor device
- 320:: signal processing unit

## Claims

1. A wireless sensor device, comprising:
a sensor unit that generates a detection signal based on a transmission signal emitted to a detection target and a reflection signal from the detection target of the transmission signal; and
a signal processing unit that performs signal processing on the detection signal generated by the sensor unit,
wherein the sensor unit generates a first detection signal corresponding to a first phase state, which is a phase state of the transmission signal or the reflection signal, and a second detection signal corresponding to a second phase state in which a phase of the transmission signal or the reflection signal is different from that in the first phase state, and
the signal processing unit detects phase information based on the first detection signal and the second detection signal, and performs predetermined signal processing based on the detected phase information

2. The wireless sensor device according to claim 1,
wherein the second phase state is a phase state in which the phase of the reflection signal is different by π/2 (radian) from that in the first phase state, and
the signal processing unit calculates a phase angle in orthogonal coordinates based on the first detection signal and the second detection signal, and corrects a phase of a signal based on the calculated phase angle

3. The wireless sensor device according to claim 2,
wherein the second phase state is a phase state in which the phase of the reflection signal is delayed by π/2 (radian) with respect to that in the first phase state, and
the signal processing unit calculates the phase angle as an arc tangent function for a value obtained by dividing first numerical information by second numerical information using the first numerical information corresponding to a mixed signal of the first detection signal and the second detection signal and the second numerical information corresponding to a mixed signal of the first detection signal and the first detection signal

4. The wireless sensor device according to claim 2,
wherein the second phase state is a phase state in which the phase of the reflection signal is delayed by π/2 (radian) with respect to that in the first phase state, and
the signal processing unit calculates the phase angle as an arc tangent function for a value obtained by dividing third numerical information by first numerical information using the first numerical information corresponding to a mixed signal of the first detection signal and the second detection signal and the third numerical information corresponding to a mixed signal of the second detection signal and the second detection signal

5. The wireless sensor device according to claim 2,
wherein the second phase state is a phase state in which the phase of the reflection signal is delayed by π/2 (radian) with respect to that in the first phase state, and
the signal processing unit calculates a first calculation value of the phase angle as an arc tangent function for a value obtained by dividing first numerical information by second numerical information and calculates a second calculation value of the phase angle as an arc tangent function for a value obtained by dividing third numerical information by the first numerical information using the first numerical information corresponding to a mixed signal of the first output signal and the second output signal, the second numerical information corresponding to a mixed signal of the first output signal and the first output signal, and the third numerical information corresponding to a mixed signal of the second output signal and the second output signal, and sets one value selected from the first and second calculation values or an average value of the first and second calculation values as a calculation value of the phase angle

6. The wireless sensor device according to any one of claims 1 to 5,
wherein the sensor unit includes:
a transmitting and receiving antenna for emitting the transmission signal and receiving the reflection signal;
a signal generating circuit that generates the transmission signal;
a phase shifter that generates the first and second phase states;
a first detection circuit to which a part of the transmission signal and the reflection signal corresponding to the first phase state are input; and
a second detection circuit to which a part of the transmission signal and the reflection signal corresponding to the second phase state are input, and
the signal processing unit includes:
a phase detection circuit to which the first detection signal and the second detection signal are input; and
a signal processing circuit to which the first detection signal, the second detection signal, and the phase information are input

7. The wireless sensor device according to claim 6,
wherein a plurality of the signal processing units are provided,
the signal processing unit includes a first filter, which has a predetermined pass band and to which the first detection signal is input, and a second filter, which has the predetermined pass band and to which the second detection signal is input, and
the predetermined pass band is a different band for each of the signal processing units.
